# FASCICULE DE BREVET EUROPEEN

(11) **EP 0 687 661 B1**
(45) Date de publication et mention de la délivrance du brevet: **03.06.1998**
(21) Numéro de dépôt: 94109355.1
(22) Date de dépôt: 17.06.1994
(51) Int. Cl.: C07C 39/17

(54) **Phénylindanes, procédé de préparation et utilisation**
Phenylindane, Verfahren zur Herstellung und ihre Verwendung
Phenylindanes, method of preparation and use

(43) Date de publication de la demande: 20.12.1995
(73) Titulaire: SOCIETE DES PRODUITS NESTLE S.A., 1800 Vevey (CH)
(72) Inventeur: Stadler, Richard, CH-1800 Vevey (CH)
(74) Mandataire: Archambault, Jean

(56) Documents cités:
- FR-A- 1 167 626
- COLLOQUE SCIENTIFIQUE INTERNATIONAL SUR LE CAFE(COMPTES RENDUS)(CICRD8), vol.15, no.2, 1993 pages 426 - 432 R TURESKY ET AL 'THE PRO- AND ANTIOXIDATIVE EFFECTS OF COFFEE AND ITS IMPACT ON HEALTH'

## Description

La présente invention concerne deux composés chimiques nouveaux de la famille des indanes.

On a constaté qu'il était possible d'obtenir, à partir de substances naturellement présentes dans le café, des composés nouveaux qui présentaient, de manière surprenante, des propriétés antioxydantes.

La présente invention a donc pour premier objet le trans-1-3-dihydroxy-5,6-(dihydroxyphényl-3',4')-3-méthyl-1-indane, de formule chimique I, appelé dans la suite de la description "composé trans".

La présente invention a également pour objet le cis-1-3-dihydroxy-5,6-(dihydroxyphényl-3',4')-3-méthyl-1-indane, de formule chimique II, appelé dans la suite de la description "composé cis".

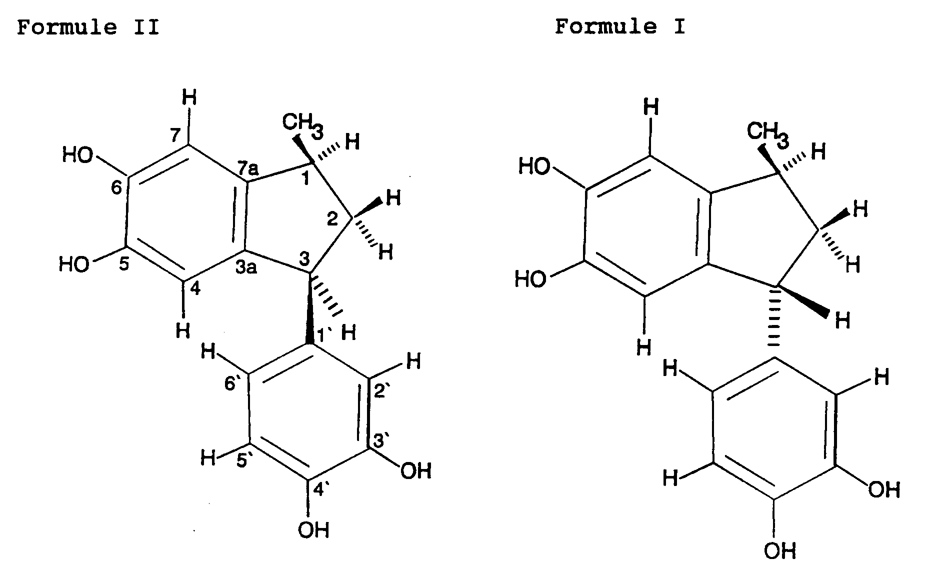

Un autre objet de l'invention est un procédé de préparation desdits composés de formule I et II par pyrolyse de l'acide caféique. Ce procédé permet l'obtention des composés cis et trans en mélange, mais également sous une forme purifiée.

On a constaté que les composés cis et trans selon la présente invention présentaient certaines propriétés antioxydantes. Ils peuvent alors être utilisés dans l'industrie agro-alimentaire et/ou dans l'industrie cosmétique comme agents antioxydants. La présente invention concerne donc également l'utilisation des composés cis et trans comme antioxydant. On peut, par exemple, ajouter les composés cis et/ou trans à raison de 5-50 ppm, dans un produit alimentaire ou dans une composition cosmétique, de manière à les protéger de l'oxydation.

On a également constaté que ces composés présentaient une certaine activité antimutagénique. La présente invention concerne donc également l'utilisation des composés cis et trans comme agent anti-mutagène.

L'invention est illustrée plus en détail dans les exemples suivants. Les exemples 1, 2 et 3 décrivent trois procédés de préparation des composés cis et trans, l'exemple 4 décrit les tests permettant d'identifier leur structure, les exemples 5 et 6 illustrent leurs propriétés antioxydantes, l'exemple 7 décrit une application alimentaire, les exemples 8 à 12 décrivent des applications cosmétiques, et les exemples 13 à 15 illustrent les propriétés anti-mutagènes de ces composés.

### Exemple 1

On mélange 1,0 g d'acide caféique (5,55 mmoles) à 30 ml d'acide sulfurique 1 M, et l'on chauffe le mélange qu'on laisse sous reflux pendant environ 2 heures. On obtient une solution jaune claire que l'on laisse refroidir jusqu'à température ambiante avant de l'extraire à l'éther. On récupère la phase éthérée que l'on sèche sur sulfate de sodium, puis que l'on filtre et concentre sous pression réduite à une température de 40°C. On obtient ainsi 0,85 g d'une substance gommeuse.

On effectue une chromatographie sur couche mince de ladite substance, sur gel de silice (Merck, 60-254, 0,5 mm) avec de l'éther comme solvant (Rf=0,75, visualisation par UV à 256 nm. On obtient ainsi 93 mg (0,34 mmoles) d'un mélange comprenant les deux composés cis et trans.

On effectue une chromatographie liquide haute pression (HPLC) de 74 mg du mélange, de manière à séparer les deux isomères cis et trans. La chromatographie est effectuée dans les conditions suivantes.
- colonne: Supelco LC-18 DB (10 x 250 mm).
- solvant: acétate d'ammonium, pH 4.
- éluants: on utilise pendant les 20 premières minutes une solution à 55% d'acide acétique, et 45% d'acétate d'ammonium. Puis pendant les 20 minutes suivantes, on utilise une solution à 55% de méthanol, et 45% d'acétate d'ammonium.
- les pics sont détectés par photométrie

Le composé trans est élué le premier, avec un temps de rétention de 33,8 minutes; le composé cis est élué en second, avec un temps de rétention de 36,1 minutes.

On obtient ainsi, après lyophilisation, 22 mg de composé trans et 23 mg de composé cis (rendement de 5-8%).

Le composé cis contient toutefois une impureté qu'il est possible d'éliminer par chromatographie sur colonne Sephadex LH20, de la manière suivante. Un mélange de 20 mg du composé cis impur dans 4 ml d'acide chlorhydrique 10 mM, est introduit dans une colonne Sephadex LH20 pré-équilibrée avec HCl 10 mM. On récupère des fractions de 5 ml chacune. La colonne est éluée avec premièrement 500 ml d'HCl 10 mM, puis deuxièmement 500 ml d'un mélange à 20% de méthanol dans HCL 10 mM, et enfin avec troisièmement 500 ml d'un mélange à 50% de méthanol dans HCl 10 mM. Le composé cis pur est élué après environ 300 ml de fraction c), puis est séché sous pression réduite à 35°C et lyophilisé. On obtient 10 mg du composé cis sous forme purifiée.

### Exemple 2

On chauffe 8 mg d'acide caféique à 226°C, sous une pression de 0,02-0,05 bar, pendant environ 15-20 minutes. Avant la pyrolyse, on ajoute 30 µl de tampon potassium-phosphate 10 mM à pH 6,0, on enlève l'eau en excès par application d'une pression réduite, puis on chauffe le mélange dans un bloc thermostaté à 226-228°C pendant 15 minutes. On obtient ainsi un composé huileux comprenant un mélange des deux composés cis et trans.

On effectue une chromatographie sur couche mince, puis une chromatographie liquide haute pression, de la même manière que dans l'exemple 1, et l'on récupère les deux isomères de façon isolée, avec un rendement de 5 à 8 %.

### Exemple 3

On prépare un mélange de 2 g d'acide caféique (11,11 mmoles) dans 4-5 ml d'eau. On lui applique une pression de 0,08-0,3 mbar tout en chauffant à 70-75°C, de manière à faciliter l'évaporation de l'eau en excès. Lorsqu'il n'y a plus d'eau en excès, on chauffe jusqu'à environ 230-235°C sous pression réduite, pendant environ 25 minutes. L'acide caféique alors brunit et forme une huile foncée. Lorsque le chauffage est terminé, on laisse le mélange se refroidir jusqu'à température ambiante, sous pression réduite, puis on dissout le résidu dans 10 ml de méthanol acide (contenant quelques gouttes d'HCl 10 mM). Le résidu est alors séché sous pression réduite à 35°C, puis récupéré dans 5 ml d'éther, purifié sur gel de silice à l'aide d'une colonne pré-équilibrée avec de l'éther, à une vitesse de 20 ml/min.

Les composés cis et trans sont élués les premiers (après environ 200 ml d'éther rajouté), puis séchés sous pression réduite, à 35°C, de manière à obtenir 80 mg (0,29 mmoles) d'une huile rougeâtre contenant une quantité égale de composés cis et trans, d'où un rendement de 4,2%.

### Exemple 4

### 1) Spectres de masse

Le spectre de masse du composé trans, effectué dans le perfluoro-kérosène, donne une valeur de 272,10507 daltons, la valeur théorique pour un composé de formule C16H1604 étant de 272,1048 daltons. Le spectre de masse du composé cis, effectué dans le perfluoro-kérosène donne une valeur de 272,1045 daltons.

### 2) Spectres RMN dans le DMSO à 360,13 MHz

Le spectre de résonance magnétique nucléaire du proton des composés cis et trans est effectué dans le diméthylsulfoxyde (DMSO-d6) à 20,5°C, à 360,13 MHz. Il montre les signaux caractéristiques suivants:

| Proton | composé cis | | | composé trans | | |
|---|---|---|---|---|---|---|
| | sign. (ppm) | mult. | J | sign. (ppm) | mult. | J |
| 1-H | 2,951 | m | | 3,129 | 6 lignes | |
| | | | | J moyen | | 6,74 |
| 1-CH3 | 1,222 | d | 6,69 | 1,135 | d | 6,88 |
| 2-H | 1,357 | d | 12,05 | 1,961 | ddd | 12,53 |
| | | t | 10,49 | | | 8,03 |
| | | | | | | 5,64 |
| 2-H | 2,53 | | 2,066 | | ddd | 12,62 |
| | | | | | | 7,44 |
| | | | | | | 5,81 |
| 3-H | 3,872 | dd | 10,21 | 4,053 | dd s | total |
| | | | 7,11 | | br | 13,73 |
| 4-H | 6,148 | d | 0,73 | 6,280 | s | |
| 7-H | 6,575 | d | 0,73 | 6,581 | s | |
| 2'-H | 6,516 | d | 2,04 | 6,418 | d | 1,97 |
| 5'-H | 6,646 | d | 7,96 | 6,600 | d | 7,99 |
| 6'-H | 6,459 | dd | 8,03 | 6,372 | dd | 7,98 |
| | | | 2,07 | | | 2,04 |
| ar-OH | 8,743 | s br | | 8,704 | s br | |
| | 8,635 | s br | | 8,590 | s br | |
| | 8,555 | s br | | 8,582 | s br | |
| | 8,505 | s br | | 8,549 | s br | |
| Abréviations: sign.: signal, mult: multiplicité, s: singulet, d: doublet, t: triplet, m: multiplet, br: large J: constante de couplage, en Hz. | | | | | | |

### 3) Spectre RMN dans le CD3OD à 360,13 MHz

Le spectre de résonance magnétique nucléaire du proton des composés cis et trans est effectué dans CD3OD à 20,5°C, à 360,13 MHz. Il montre les signaux caractéristiques suivants:

| Proton | composé cis | | | composé trans | | |
|---|---|---|---|---|---|---|
| | sign. (ppm) | mult. | J | sign. (ppm) | mult. | J |
| 1-H | 3,002 | m | 6,75 | 3,176 | 6 lignes | |
| | | dont | | | J moyen | 6,73 |
| 1-CH3 | 1,275 | d | 6,71 | 1,187 | d | 6,91 |
| 2-H | 1,427 | dt | 12,06 | 2,040 | ddd | 12,48 |
| | | | 10,41 | | | 8,10 |
| | | | | | | 5,53 |
| 2-H | 2,575 | dt | 12,12 | 2,146 | ddd | 12,48 |
| | | | 6,98 | | | 7,61 |
| | | | | | | 5,96 |
| 3-H | 3,924 | dd | 10,50 | 4,122 | dd | 7,96 |
| | | | 7,16 | | s br | 6,04 |
| 4-H | 6,265 | d | 0,99 | 6,378 | (d) | 0,60 |
| 7-H | 6,631 | d | 1,03 | 6,637 | (d) | 0,84 |
| 2'-H | 6,593 | d | 2,05 | 6,483 | d | 2,07 |
| 5'-H | 6,692 | d | 8,02 | 6,645 | d | >7,94 |
| 6'-H | 6,530 | dd | 8,06 | 6,436 | dd | 8,09 |
| | | | 2,09 | | | 2,11 |
| Abréviations: sign.: signal, mult: multiplicité, s: singulet, d: doublet, t: triplet, m: multiplet, br: large J: constante de couplage, en Hz. | | | | | | |

### 4) Spectre RMN dans CD3OD à 90,56 Mhz

Le spectre de résonance magnétique nucléaire du carbone 13 des composés cis et trans est effectué dans CD3OD à 21°C, à 90,56 MHz. Les mesures pour les composés cis et trans sont effectuées avec le TMS comme standard. La dernière colonne du tableau correspond aux valeurs théoriques calculées à l'aide du programme STN Specinfo SPECAL. il montre les signaux caractéristiques suivants.

| Carbone | Composé cis | | Composé trans | | Calculé | |
|---|---|---|---|---|---|---|
| | sign. (ppm) | mult. | sign. (ppm) | mult. | sign. (ppm) | mult. |
| 1-CH | 39,01 | d | 38,94 | d | 37,22 | d |
| 1-CH3 | 20,15 | q | 21,32 | q | 20,18 | q |
| 2-CH2 | 48,44 | t | 46,74 | t | 42,37 | t |
| 3-CH | 50,96 | d | 50,02 | d | 54,10 | d |
| 3a-C | 139,70 | s | 138,73 | s | 135,91 | s |
| 4-CH | 112,46 | d | 112,66 | d | 117,52 | d |
| 5-C-OH | 144,94 | s | 145,14 | s | 144,86 | s |
| 6-C-OH | 145,23 | s | 145,44 | s | 144,86 | s |
| 7-CH | 110,57 | d | 110,99 | d | 117,52 | d |
| 7a-C | 141,12 | s | 141,47 | s | 151,28 | s |
| 1'-C | 138,69 | s | 139,58 | s | 135,41 | s |
| 2'-CH | 116,15 | d | 115,72 | d | 116,04 | d |
| 3'-C-OH | 146,21 | s | 146,12 | s | 144,81 | s |
| 4'-C-OH | 144,60 | s | 144,38 | s | 146,03 | s |
| 5'CH | 116,15 | d | 116,13 | d | 116,88 | d |
| 6'CH | 120,70 | d | 120,11 | d | 120,89 | d |
| Abréviations: sign.: signal, mult: multiplicité, s: singulet, d: doublet, t: triplet, m: multiplet, br: large, q: quadruplet J: constante de couplage, en Hz. | | | | | | |

### 5) Spectres UV

Les spectres UV des composés cis et trans, effectués dans HCl 10 mM, à l'aide d'un spectrophotomètre Perkin-Elmer Lamda 5, montrent un pic à λ = 285,5 nm pour le composé cis et un pic à λ = 285,5 nm pour le composé trans.

On peut déduire de tous ces tests que les composés cis et trans correspondent bien à la structure illustrée aux figures I et II.

### Exemple 5

### 1) Préparation des composés cis et trans

On mélange 500 µl d'une solution aqueuse de linoléate d'éthyle à 1,5 mg/ml, à 4% d'une solution aqueuse de sulfate de dodecyl-sodium. On ajoute 0,25 mmol d'un tampon Tris/HCl (pH 6,5), 1 µmol de FeCl2,4H2O, 0,5 µmol de H2O2 et une certaine quantité du composé à tester, selon le tableau ci-dessous. On complète à 1 ml avec de l'eau distillée. On obtient ainsi une émulsion.

On prépare également des contrôles positifs ne contenant pas de composé à tester, et des contrôles négatifs ne contenant ni H2O2, ni FeCl2.

Les différents échantillons sont incubés, dans l'obscurité, à 37°C, pendant 16 heures, tout en étant continuellement agités à 190 tours/minute. On ajoute alors 20 µl d'une solution d'hydroxytoluène butylé à 4%. On obtient ainsi des échantillons adéquats prêts à être testés.

### 2) Préparation du MDA (malondialdéhyde)standard

On effectue une hydrolyse acide en mélangeant 306 mg de dérivé bisdiethyle à 50 ml d'acide chlorhydrique 0,1 N, sous agitation, à 40°C, pendant 30 minutes. On refroidit la solution jusqu'à température ambiante puis on la dilue avec HCl 0,1 M jusqu'à un volume final de 100 ml. On obtient 100 mg de MDA libre dans 100 ml d'HCl 0,1 N.

### 3) Méthode photométrique

On prépare un mélange contenant 0,2 ml de solution tampon (2M, pH 3,5), 0,2 ml d'une solution aqueuse d'acide thiobarbiturique à 1,6%, 0,05 ml d'une solution à 8% de sulfate de dodecyl-sodium et 0,05-0,1 ml de l'échantillon à tester préparé tel que précédemment. On chauffe ce mélange à 90°C, au bain-marie, pendant 60 minutes, puis on laisse refroidir jusqu'à température ambiante. On ajoute 1 ml d'une solution aqueuse saturée de n-butanol. Le mélange est agité pendant 5 minutes puis centrifugé à 14000 tours/minute pendant 5-10 minutes. On récupère la phase organique et l'on en effectue une mesure photométrique à 532 nm.

### 3) Analyse par HPLC

Il est également possible de mesurer la quantité de MDA formé par HPLC (chromatographie liquide, haute pression). Dans ce cas, on dilue 2 parties en volume de la phase organique récupérée plus haut, dans 1 partie en volume d'un mélange 2/1, en volume, n-butanol/méthanol. La séparation est effectuée à l'aide d'une colonne de chromatographie, dans les conditions suivantes:
- colonne: Supelco LC-18 DB (4,6 x 250 mm)
- solvant: mélange 50/50, en poids/volume, eau/méthanol contenant 0,05%, en poids/volume, de sulfate de tétrabutylammonium.

Des échantillons de 0,02 ml sont injectés dans un chromatographe équipé avec un détecteur de photodiode et d'un fluoromètre. La détection est effectuée dans les conditions suivantes.
- longueurs d'ondes pour la détection photométrique: 267 nm et 532 nm
- détection fluorométrique: λex = 515 nm et λem = 545 nm
- slit width: 2 x 2 mm
- filtre cut-off: 520 nm
- temps de réponse :4 s
- fréquence du flash: 110 Hz
- temps de rétention de 5,3 minutes

### 5) résultats

On détermine l'activité antioxydante des composés cis et trans, ainsi que celle, pour comparaison, de l'α-tocophérol, à pH 6,5. La quantité de MDA formée est calculée par mg de linoléate d'éthyle et quantifié par HPLC avec détection fluorométrique. On obtient les résultats suivants:

| antioxydants | MDA formé (nmoles) | | |
|---|---|---|---|
| | 0 | 10 µg/ml | 50 µg/ml |
| composé cis | 28 | 5 | 14 |
| composé trans | 27 | 4 | 18 |
| α-tocophérol | 28 | 21 | 10 |

On constate donc que les deux composés cis et trans présentent une activité antioxydante égale voire supérieure, à celle présentée par l'α-tocophérol, pour une concentration allant au moins jusqu'à 50 µg/ml.

A concentration plus élevée, l'effet devient inverse, c'est-à-dire pro-oxydant, ce qui peut être dû à une production plus élevée de peroxyde d'hydrogène durant l'autooxydation.

Le tableau suivant donne l'activité antioxydante de divers composés, à plusieurs concentrations. La quantité de MDA formé est une moyenne entre les valeurs trouvées par détection photométrique et par HPLC/fluorométrie.

| Composes | conc (µM) | MDA formé (nmoles/mg) |
|---|---|---|
| α-tocophérol | 20 | 6,87 |
| α-tocophérol | 100 | 3,55 |
| α-tocophérol | 200 | 2,79 |
| composé trans | 20 | 4,45 |
| composé trans | 100 | 7,63 |
| composé trans | 200 | 16,95 |
| composé cis | 20 | 4,52 |
| composé cis | 100 | 8,98 |
| composé cis | 200 | 20,33 |
| pyrocatechol | 20 | 4,96 |
| pyrocatechol | 100 | 10,34 |
| pyrocatechol | 200 | 19,24 |
| 4-ethylpyrocatechol | 20 | 5,53 |
| 4-ethylpyrocatechol | 100 | 3,70 |
| 4-ethylpyrocatechol | 200 | 5,77 |
| acide caféique | 20 | 6,65 |
| acide caféique | 100 | 11,79 |
| acide caféique | 200 | 20,1 |
| thymol | 20 | 2,13 |
| thymol | 100 | 1,01 |
| thymol | 200 | 0,91 |
| sesamol | 20 | 3,41 |
| sesamol | 100 | 1,45 |
| sesamol | 200 | 2,84 |
| quercitrin | 20 | 6,23 |
| quercitrin | 100 | 10,96 |
| quercitrin | 200 | 18,23 |
| acide ascorbique | 20 | 8,83 |
| acide ascorbique | 100 | 6,86 |
| acide ascorbique | 200 | 5,60 |

La quantité de MDA formé est donnée en nmoles par mg de linoléate d'éthyle.

On constate à nouveau que pour une concentration de 20 µM, les composés cis et trans présentent une activité égale voire supérieure, à celles d'antioxydants connus. Cette activité reste correcte pour une concentration de 100 µM, mais diminue énormément à concentration plus élevée, de la même manière que celle d'antioxydants connus. Cela semble donc bien dû à la méthode employée.

### Exemple 6

On incorpore 500 mg/kg d'un mélange des composés cis et trans, préparé selon l'exemple 3, à une huile de tournesol comprenant en outre 10000 mg/kg de phospholipides de soja, au moyen du procédé faisant l'objet de la demande de brevet parallèle de la titulaire, déposée ce jour, sous le titre: "Incorporation dans un lipide d'un principe actif hydrosoluble". L'huile ainsi stabilisée est ensuite filtrée, et l'activité est mesurée avec le test classique d'oxydation accélérée Rancimat à 110°C, décrit dans EP 326829, par exemple.

Pour comparaison, on réalise le même test Rancimat avec la solution d'huile de tournesol seule, également avec la solution d'huile de tournesol comprenant 10000/kg de phospholipides de soja, et également avec cette dernière solution comprenant 1000 mg/kg de vitamine C.

| Essais | phospholipides (mg/kg) | Vit. C (mg/kg) | phénylindanes de l'exemple 3 (mg/kg) | OSI 110°C (h) | PF |
|---|---|---|---|---|---|
| 1 | - | - | - | 5,0 | 1,0 |
| 2 | 10000 | - | - | 7,0 | 1,75 |
| 3 | 10000 | 1000 | - | 20,5 | 4,1 |
| 4 | 10000 | - | 500 | 30,5 | 6,1 |

Les temps d'induction obtenus, exprimés en h, représentent les valeurs OSI (Oil Stability Index). Enfin, PF est le rapport OSI de l'huile stabilisée par le procédé décrit précédemment/OSI de l'huile non stabilisée, qui représente l'augmentation du temps d'induction.

Les phénylindanes selon la présente invention démontrent ainsi une excellente protection antioxydante, qui, comparée à celle de la vitamine C, lui est nettement supérieure. Il ressort, en effet, que l'huile comprenant les composés cis et trans selon la présente invention, reste stable environ 4 fois plus longtemps que l'huile sans additif, et environ 1,5 fois plus longtemps que l'huile comprenant la vit. C.

### Exemple 7

On prépare une huile selon l'exemple 6, destinée à assaisonner une salade de légumes frais sans vinaigre, et comprenant 989 g d'huile de tournesol, 10 g de phospholipides, et 1 g d'un mélange des composés cis et trans préparé comme décrit à l'exemple 3.

Une assiette de légume frais de goût neutre peut être agrémenté avec 10 g de l'huile précédente, par exemple, et apporter ainsi 10 mg de phénylindanes selon la présente invention.

### Exemple 8

Cet exemple conçerne la préparation d'une composition cosmétique de soin de la peau, par exemple, contenant des lipides, dont la partie lipidique est protégée contre l'oxydation par la présence des antioxydants selon la présente invention.

On prépare ainsi 77 ml d'une solution tampon à pH 5,7 (à base de phosphate de sodium), on y ajoute 10 mg de chitosane dissout dans de l'acide acétique dilué, présentant un poids moléculaire de environ 180000, un degré de déacétylation de environ 85% et provenant de crevettes, puis on ajoute ensuite 8 g de glycérol et le mélange est chauffé au bain-marie à 80°C.

On y ajoute alors 3 g d'huile de tournesol préalablement chauffée à 80°C, préparée selon l'exemple 6, comprennant 3 mg d'un mélange des composés cis et trans anti-oxydants selon l'exemple 3, et 30 mg de phospholipides de soja.

Parallèlement, on fait fondre 12 g de stéarate de glycérol à 80°C, qu'on ajoute lentement au mélange précédent, sous agitation. On laisse le mélange se refroidir jusqu'à température ambiante, en l'homogénéisant de temps à autre.

On obtient ainsi une crème de soin contenant 30 µg/g de phénylindanes selon la présente invention.

### Exemples 9-12

Ces exemples conçernent la préparation de compositions cosmétiques de soin de la peau contenant des lipides dont la partie lipidique est protégée contre l'oxydation par addition dans la phase lipidique d'un mélange des anti-oxydants selon la présente invention, rendus préalablement liposoluble comme décrit à l'exemple 6. Les pourcentages sont donnés en poids.

Pour fabriquer ces émulsions, on mélange les composants de la phase lipidique A et on la chauffe à 75°C. On prépare la phase aqueuse B, et on la chauffe également à 75°C, puis on l'ajoute à la phase lipidique A en brassant lentement, et on refroidit ensuite sous brassge lent jusqu'à température ambiante, soit environ 25°C. A cette température, on ajoute lentement le cas échéant les constituants C dans l'ordre de la formule.

Pour fabriquer le produit anhydre de l'exemple 12, on mélange tous les constituants à froid, suivant l'ordre de la formule sous brassage lent.

| **9. Crème hydratante (émulsion huile-dans-eau)** **Phase lipidique A** | |
|---|---|
| | **%** |
| Peg-6-stéarate, glycérate et peg-20-céthyl éther (peg:polyéthylène glycol) | 15 |
| Huile de vaseline | 5 |
| Huile de germe de blé stabilisée selon l'ex. 6, avec 0,1% du mélange de composés cis et trans antioxydants selon l'ex.3, et 1% de phospholipides de soja | 3 |
| Huiles d'amandes douces | 2 |
| Alcool cétylique | 1 |
| Isostéaryl-isostéarate | 2 |
| 2-Octyl-docécyl-mystirate | 1 |
| Cire de lanoline | 1 |

| **Phase aqueuse B** | |
|---|---|
| | **%** |
| Méthylisothiazoline | 0,1 |
| Eau déminéralisée | 59,6 |
| Protéine de placenta humain | 2 |

| **Additifs C** | |
|---|---|
| | **%** |
| Propylèneglycol et extrait de calendula | 2 |
| Collagène soluble (2) dans de l'eau déminéralisée (4) | 6 |
| Parfum | 0,3 |

| **10. Crème anti-rides (émulsion huile-dans-eau)** **Phase lipidique A** | |
|---|---|
| | **%** |
| Cire d'abeille hydrophile non ionique | 10 |
| Huile de vaseline | 4 |
| Huile d'amande douce stabilisée selon l'ex. 6, avec 0,1% du mélange de composés cis et trans antioxydants selon l'ex. 3, et 1% de phospholipides de soja | 3 |
| Isostéaryl-isostéarate | 5 |
| Ethyl-linoléate | 1 |
| Huile minérale, huile de noyau d'abricots et extrait de calendula | 4 |
| Huile de noyaux d'abricots | 3 |

| **Phase aqueuse B** | |
|---|---|
| | **%** |
| Méthylisothiazole | 0,1 |
| Eau déminéralisée | 64,7 |
| Carbopol 934 (polymère d'acide acrylique polyréticulé) | 0,3 |
| Triéthanolamine | 0,6 |
| Propylène glycol et extrait de sureau | 2 |
| Collagène hydrosoluble | 2 |
| Parfum | 0,3 |

| **11. Crème pour bébés (émulsion huile-dans-eau)** **Phase lipidique A** | |
|---|---|
| | **%** |
| Polyéthylène glycol-6-32-stéarate | 10 |
| Huile d'amande douce stabilisée selon l'ex. 6, avec 0,1% du mélange de composés cis et trans antioxydants selon l'ex. 3, et 1% de phospholipides de soja | 6 |
| Huile de vaseline | 4 |
| Huile de palmiste, huile de palme et peg 6 | 3 |
| Acide stéarique | 1 |

| **Phase aqueuse B** | |
|---|---|
| | **%** |
| Méthylisothiazoline | 1 |
| Eau déminéralisée | 67,5 |
| Glycérine | 3 |
| Propylène glycol et extrait de calendula | 4 |
| Parfum | 0,5 |

| **12. Huile satinée (anhydre)** | |
|---|---|
| | **%** |
| Huile de tournesol selon l'ex. 6, comprenant 0,1% du mélange de composés cis et trans antioxydants selon l'ex. 3, et 1% de phospholipides de soja | 3 |
| Triglycérides d'acides caprylique et caprique | 30 |
| Diméthyl polysiloxane cyclique | 25,7 |
| Propylèneglycol-Diperlagonate | 37,8 |
| Octyl méthoxycinnamate | 3 |
| Parfum | 0,5 |

### Exemples 13-15

On mesure l'activité antimutagène du mélange des composés cis et trans, préparé comme décrit à l'exemple 3, selon le test décrit dans Maron et al. (Mutat. Resarch, 113, p 173-215,1983). Pour cela, on cultive en boite les cellules bactériennes décrites dans Maron et al., pendant 7 h sous aération, on ajoute 1 µmole d'un agent mutagène, à savoir du t-butyl-hydroperoxyde (tBOOH), à 0,1 ml des bactéries cultivées en présence de 35 à 3500 µg/boite de phénylindanes selon la présente invention, préparées soit en mélange selon l'exemple 3, soit de façon purifiées selon l'exemple 1. On incube finalement les boites pendant 72 h à 37°C, puis on mesure le nombre de révertants.

Pour comparaison, on mesure le nombre de révertants sur des boites qui non pas été soumises à l'agent mutagène. Les résultats sont présentés dans le tableau ci-après.

| Exemples 13-15 | Phénylindanes (µg/boite) | Cfu (A) de révertants + tBOOH | Cfu (B) de révertants -tBOOH | A-B |
|---|---|---|---|---|
| Ex. 13 Mélange des composés cis et trans | 0,0 | 961 | 304 | 657 |
| | 35 | 843 | 333 | 510 |
| | 70 | 816 | 342 | 474 |
| | 140 | 651 | 362 | 289 |
| | 280 | 512 | 361 | 151 |
| | 500 | 427 | 298 | 129 |
| | 1000 | 508 | 226 | 282 |
| | 3500 | 3 | 3,5 | - |
| Ex. 14 composé cis | 70 | 812 | 340 | 472 |
| | 140 | 655 | 340 | 315 |
| | 500 | 420 | 305 | 115 |
| Ex. 15 composé trans | 70 | 827 | 320 | 507 |
| | 140 | 660 | 348 | 312 |
| | 500 | 432 | 289 | 143 |

Comme on peut le voir dans le tableau ci-dessus, une concentration supérieure à 1 µg/boite de phénylindanes selon la présente invention, présente un effet bactéricide.

De plus, une concentration de l'ordre de 140 µg est capable de réduire de plus de 50% le nombre de révertants induits pas l'agent mutagène. Les composé cis et trans présentent donc, une activité anti-mutagène marqué, seuls ou en combinaison.

## Revendications

1. Trans-1-3-dihydroxy-5,6-(dihydroxyphényl-3',4')-3-méthyl-1-indane.

2. Cis-1-3-dihydroxy-5,6-(dihydroxyphényl-3',4')-3-méthyl-1-indane.

3. Procédé de préparation des composés trans-1-3-dihydroxy-5,6-(dihydroxyphényl-3',4')-3-méthyl-1-indane et cis-1-3-dihydroxy-5,6-(dihydroxyphényl-3',4')-3-méthyl-1-indane par pyrolyse de l'acide caféique.

4. Procédé permettant d'éviter l'oxydation de composés alimentaires ou cosmétiques consistant à leur ajouter le composé trans-1-3-dihydroxy-5,6-(dihydroxyphényl-3',4')-3-méthyl-1-indane.

5. Procédé permettant d'éviter l'oxydation de composés alimentaires ou cosmétiques consistant à leur ajouter le composé cis-1-3-dihydroxy-5,6-(dihydroxyphényl-3',4')-3-méthyl-1-indane.

6. Utilisation du composé trans-1-3-dihydroxy-5,6-(dihydroxyphényl-3',4')-3-méthyl-1-indane comme antioxydant.

7. Utilisation du composé cis-1-3-dihydroxy-5,6-(dihydroxyphényl-3',4')-3-méthyl-1-indane comme antioxydant.

8. Utilisation du composé trans-1-3-dihydroxy-5,6-(dihydroxyphényl-3',4')-3-méthyl-1-indane comme agent anti-mutagène.

9. Utilisation du composé cis-1-3-dihydroxy-5,6-(dihydroxyphényl-3',4')-3-méthyl-1-indane comme agent anti-mutagène.

## Claims

1. *Trans*-1-methyl-3-(dihydroxyphenyl-3',4')-5,6-dihydroxy indane.

2. *Cis*-1-methyl-3-(dihydroxyphenyl-3',4')-5,6-dihydroxy indane.

3. Process for the preparation of the compounds *trans*-1-methyl-3-(dihydroxyphenyl-3',4')-5,6-dihydroxy indane and *cis*-1-methyl-3-(dihydroxyphenyl-3',4')-5,6-dihydroxy indane by the pyrolysis of caffeic acid.

4. Process enabling the oxidation of food or cosmetic compositions to be prevented, comprising the addition thereto of the compound *trans*-1-methyl-3-(dihydroxyphenyl-3',4')-5,6-dihydroxy indane.

5. Process enabling the oxidation of food or cosmetic compositions to be prevented, comprising the addition thereto of the compound *cis*-1-methyl-3-(dihydroxyphenyl-3',4')-5,6-dihydroxy indane.

6. Use of the compound *trans*-1-methyl-3-(dihydroxyphenyl-3',4')-5,6-dihydroxy indane as an antioxidant.

7. Use of the compound *ci*s-1-methyl-3-(dihydroxyphenyl-3',4')-5,6-dihydroxy indane as an antioxidant.

8. Use of the compound *trans*-1-methyl-3-(dihydroxyphenyl-3',4')-5,6-dihydroxy indane as an antimutagenic agent.

9. Use of the compound *cis*-1-methyl-3-(dihydroxyphenyl-3',4')-5,6-dihydroxy indane as an antimutagenic agent.

## Patentansprüche

1. *1,3-trans*-5,6-Dihydroxy-3-(3',4'-dihydroxyphenyl)-1-methyl-indan.

2. *1,3-cis*-5,6-Dihydroxy-3-(3',4'-dihydroxyphenyl)-1-methyl-indan.

3. Verfahren zur Herstellung der Verbindungen *1,3-trans*-5,6-Dihydroxy-3-(3',4'-dihydroxyphenyl)-1-methyl-indan und *1,3-cis*-5,6-Dihydroxy-3-(3',4'-dihydroxyphenyl)-1-methyl-indan durch Pyrolyse von Kaffeesäure.

4. Verfahren, das die Oxidation von Nahrungsmittel- oder Kosmetikzusammensetzungen vermeidet, das darin besteht, daß man ihnen die Verbindung *1,3-trans*-5,6-Dihydroxy-3-(3',4'-dihydroxyphenyl)-1-methyl-indan zugibt.

5. Verfahren, das die Oxidation von Nahrungsmittel- oder Kosmetikzusammensetzungen vermeidet, das darin besteht, daß man ihnen die Verbindung *1,3-cis*-5,6-Dihydroxy-3-(3',4'-dihydroxyphenyl)-1-methyl-indan zugibt.

6. Verwendung der Verbindung *1,3-trans*-5,6-Dihydroxy-3-(3',4'-dihydroxyphenyl)-1-methyl-indan als Antioxidans.

7. Verwendung der Verbindung *1,3-cis*-5,6-Dihydroxy-3-(3',4'-dihydroxyphenyl)-1-methyl-indan als Antioxidans.

8. Verwendung der Verbindung *1,3-trans*-5,6-Dihydroxy-3-(3',4'-dihydroxyphenyl)-1-methyl-indan als antimutagenes Mittel.

9. Verwendung der Verbindung *1,3-cis*-5,6-Dihydroxy-3-(3',4'-dihydroxyphenyl)-1-methyl-indan als antimutagenes Mittel.
